# FASCICULE DE BREVET EUROPEEN

(11) **EP 3 684 287 B1**
(45) Date de publication et mention de la délivrance du brevet: **19.07.2023**
(21) Numéro de dépôt: 18769219.9
(22) Date de dépôt: 20.09.2018
(51) Int. Cl.: A61B 90/00

(54) **DISPOSITIF DE GUIDAGE D'INSTRUMENT**
INSTRUMENTENFÜHRUNGSVORRICHTUNG
INSTRUMENT GUIDING DEVICE

(30) Priorité: 22.09.2017 FR 1758795
(43) Date de publication de la demande: 29.07.2020
(73) Titulaire: Koelis, 38240 Meylan (FR)
(72) Inventeur: BAUMANN, Michael, 38700 LA TRONCHE (FR); GAUDARD, Eric, 69002 Lyon (FR); HENRI, Patrick, 92270 Bois-Colombes (FR); LEROY, Antoine, 78390 Bois D'Arcy (FR); MIGNON, Paul, 60003 Lyon (FR)
(74) Mandataire: Decorchemont, Audrey Véronique Christèle
(86) Numéro de dépôt international: PCT/EP2018/075505
(87) Numéro de publication internationale: WO 2019/057833

(56) Documents cités:
- WO-A1-2007/095637
- WO-A1-2016/184746
- US-A1- 2001 029 334
- US-A1- 2011 234 780
- US-A1- 2013 211 244
- US-B1- 6 256 529
- US-B1- 6 733 458

## Description

L'invention concerne un dispositif de guidage d'instrument.

### ARRIERE PLAN TECHNOLOGIQUE DE L'INVENTION

Un grand nombre d'opérations est basé sur l'insertion d'un instrument dans le corps du patient à travers un orifice, naturel ou artificiel. C'est le cas par exemple d'une biopsie avec l'insertion d'une aiguille (pour le prélèvement d'échantillons), d'une curiethérapie avec l'insertion d'un cathéter (pour le positionnement de grains radioactifs), ou encore d'autres thérapies ciblées telles que la cryothérapie ou la thérapie laser.

Usuellement, on associe ainsi un système d'imagerie médicale à l'instrument afin de contrôler sa position dans le corps du patient.

Dans le cas d'une biopsie de la prostate, il est ainsi connu un dispositif comprenant un porte-aiguille associé à une sonde d'imagerie médicale.

Dans le cas d'une approche transrectal, le porte-aiguille est solidaire de la sonde et est introduit avec elle dans le rectum du patient. Le porte-aiguille ne présente ainsi qu'un seul orifice à travers lequel guider l'aiguille à biopsie lorsque le porte-aiguille est en place contre la prostate.

Il s'avère donc relativement simple pour un opérateur de diriger l'aiguille correctement notamment du fait que le porte-aiguille est à proximité immédiate de la prostate.

En revanche lors d'un abord transpérinéal, le guidage de l'aiguille s'avère bien plus difficile. En effet, avec cette approche, seule la sonde est encore introduite dans le rectum : le porte-aiguille demeure hors du patient et posé contre la peau du périnée du patient. L'aiguille a donc un plus grand chemin à parcourir ce qui en complexifie le pilotage. Le document WO 2016/184746 A1 divulgue un système pour estimer la trajectoire d'un instrument inséré dans le corps. Dans ce document la position de l'instrument est estimée par moyen de ultrason et imagerie à résonance magnétique.

### OBJET DE L'INVENTION

Un but de l'invention est de proposer un dispositif de guidage facilitant le pilotage d'un instrument vis-à-vis d'un patient.

Un but de l'invention est également de proposer un procédé correspondant.

### BREVE DESCRIPTION DE L'INVENTION

En vue de la réalisation de ce but, on propose un dispositif de guidage d'un instrument médical selon la revendication 1 comprenant :
- l'instrument médical,
- un appareil de contrôle comportant un support et une sonde d'imagerie médicale qui est agencée sur le support et qui est destinée à être positionnée à proximité d'au moins une cible présente dans ou sur le patient,
- un écran, et
- une unité de commande du dispositif qui est reliée à l'écran et à la sonde pour générer au moins une image en trois dimensions,

l'unité de commande étant configurée pour générer à l'écran à partir d'au moins ladite image en trois dimensions, au moins une image en deux dimensions montrant une déformation de l'instrument,
l'unité de commande étant configurée pour, à partir de cette déformation de l'instrument, estimer un trajet virtuel de l'instrument si on prolongeait son insertion jusqu'à la cible, et en déduire au moins une distance entre ledit trajet virtuel et la cible.

Ainsi en acquérant des images de l'instrument, on facilite le guidage de l'instrument, en particulier si l'instrument s'est déplacé relativement à la position d'application prévue de l'instrument sur la cible.

Ainsi, lorsque l'opérateur voit à l'écran que l'instrument s'est déformé, il peut arrêter son geste. En fonction du résultat de l'unité de commande sur la distance entre le trajet virtuel et la cible, l'opérateur peut alors retirer l'instrument pour mieux le positionner ou continuer son geste éventuellement en ajustant, en corrigeant la trajectoire de l'instrument si cela est possible.

L'invention s'avère ainsi particulièrement efficace pour aider au positionnement d'un instrument vis-à-vis d'une cible.

En particulier sans l'invention, le clinicien pourrait retirer l'instrument alors qu'il aurait pu atteindre convenablement la cible. Pire, il pourrait continuer son geste complètement dans l'espoir d'atteindre la cible et sans succès.

L'invention permet donc d'optimiser les insertions et de diminuer le temps de l'intervention. Par ailleurs, on limite ainsi un risque de déplacer l'instrument dans des endroits inappropriés. Si on se place dans l'exemple d'une biopsie de prostate on limite un risque de perçage de la vessie par un mauvais placement de l'aiguille.

Dans le cas d'une biopsie, on réduit également les risques d'infection, de saignements.

L'invention offre donc à un opérateur la possibilité d'afficher non seulement une image en trois dimensions du volume anatomique sur lequel intervenir mais également des images en deux dimensions dont au moins l'une permet d'afficher si besoin la déformation de l'instrument.

Ceci permet à un opérateur de visualiser plus aisément la relation entre l'instrument et la cible et donc d'intervenir plus efficacement sur la cible. En outre, la génération de l'image en deux dimensions se fait par travail sur l'image en trois dimensions ce qui permet de ne pas avoir à bouger le support.

Par ailleurs, on peut ainsi afficher à l'écran des images non accessibles directement par la sonde comme une image deux dimensions comprenant une projection de l'instrument.

Par « déformation de l'instrument » on entend ici aussi bien une déformation involontaire de l'instrument, qu'une déformation volontaire de l'instrument (c'est-à-dire voulue pour des questions de techniques opératoires) qu'une déformation permanente de l'instrument par exemple dans le cas où l'instrument ne s'étend pas selon une direction sensiblement rectiligne (par exemple l'instrument est courbe) étant entendu qu'un tel instrument peut alors présenter une déformation supplémentaire volontaire ou involontaire lors de son déplacement vis-à-vis de la cible. Par ailleurs la déformation peut tout aussi bien être de manière courbe que de manière oblique (entraînant alors une déviation de l'instrument) .

De manière particulière, on peut ainsi utiliser l'invention pour une biopsie par approche transpérinéale en assurant un bon guidage pour l'instrument.

Par cible on entend ici aussi bien une zone externe au patient qu'une zone interne au patient. La cible peut ainsi être à titre d'exemple non limitatif :
- une tumeur ou tout autre zone du corps du patient considérée comme maligne ou suspecte,
- un volume anatomique tel qu'un organe comme le rein, le sein, l'utérus, la thyroïde, le foie, la vessie, l'urètre ou la prostate ou une cavité comme par exemple le cul de sac de Douglas ou un organe et son environnement immédiat comme le rectum,
- une zone particulière au sein même du volume anatomique lui-même, de l'organe, de la cavité, de l'environnement immédiat de l'organe ...,
- une position planifiée de l'instrument,
- une zone particulière en surface même du volume anatomique lui-même, de l'organe, de la cavité, de l'environnement immédiat de l'organe ... ou bien en surface de la peau de l'utilisateur, d'une muqueuse, d'une zone du corps du patient considérée comme maligne ou suspecte ...

Par ailleurs si la sonde est introduire dans le corps du patient par orifice, l'orifice peut tout aussi bien être naturel qu'artificiel.

Optionnellement, l'unité de commande est configurée de manière à afficher sur une image en deux dimensions montrant une déformation de l'instrument, le trajet virtuel de l'instrument.

Selon l'invention , l'unité de commande calcule, à partir de plusieurs points de l'instrument déformé, une ligne formée par l'instrument déformé, l'unité de commande étant configurée pour, à partir de cette ligne, estimer le trajet virtuel de l'instrument.

Optionnellement, l'unité de commande active une alarme lorsque la distance dépasse un seuil donné.

Optionnellement, l'unité de commande affiche à l'écran un message pour activer l'alarme.

Optionnellement, au moins l'image en trois dimensions ou en deux dimensions est rafraîchie automatiquement.

Optionnellement, le trajet virtuel est mis à jour automatiquement et/ou la distance entre ledit trajet virtuel et la cible est mise à jour automatiquement.

De façon particulière, l'instrument est porté par l'appareil de contrôle.

De façon particulière, l'instrument comporte une aiguille à biopsie guidée par un porte-aiguille de l'appareil de contrôle.

De façon particulière, le dispositif comporte au moins deux instruments, le dispositif étant configuré de sorte que l'unité de commande génère à l'écran :
- une première image en deux dimensions incluant un point du premier instrument et une deuxième image en deux dimensions incluant un point du deuxième instrument; et/ou
- une image en deux dimensions incluant un point du premier instrument et un point du deuxième instrument.

De façon particulière, l'image en deux dimensions montrant une déformation de l'instrument comprend au moins un point d'introduction de l'instrument dans le volume anatomique défini par l'image en trois dimensions, ledit point étant appelé « point d'insertion de l'instrument ».

De façon particulière, l'image en deux dimensions montrant une déformation de l'instrument comprend au moins un point de l'instrument destiné à venir agir sur la cible, ledit point étant appelé « partie utile de l'instrument ».

De façon particulière, l'unité de commande est configurée pour générer à l'écran à partir de ladite image en trois dimensions, au moins une image en deux dimensions présentant à la fois le point d'insertion et la partie utile de l'instrument.

Selon un mode de réalisation particulier, l'unité de commande est configurée pour générer à l'écran au moins une image en deux dimensions, ladite image en deux dimensions étant issue d'une réduction de l'image volumique à un plan par calcul d'une moyenne ou d'une somme d'amplitudes autour d'un axe de l'image volumique.

De façon particulière, l'unité de commande est agencée de sorte à piloter la sonde afin d'aligner une position de référence de la sonde avec celle de l'instrument.

D'autres caractéristiques et avantages de l'invention ressortiront à la lecture de la description qui suit de modes de réalisation particuliers non limitatifs de l'invention.

### BREVE DESCRIPTION DES DESSINS

Dans la description qui suit, il sera fait référence aux figures ci-jointes, parmi lesquelles :
- la figure 1 est une vue schématique d'un dispositif de guidage selon un premier mode de réalisation de l'invention,
- la figure 2 illustre en partie l'appareil illustré à la figure 1,
- les figures 3a à 3d illustrent différents plans en deux dimensions pouvant être présentés à un opérateur par l'intermédiaire de la sonde de l'appareil illustré à la figure 2,
- la figure 4 et la figure 5a illustrent en partie l'appareil illustré à la figure 1,
- les figures 5b à 5d illustrent différents plans en deux dimensions pouvant être présentés à un opérateur par l'intermédiaire de la sonde de l'appareil illustré à la figure 4,
- la figure 6 illustre en partie l'appareil illustré à la figure 1,
- la figure 7a est un schéma illustrant les différentes étapes d'une mise en oeuvre par l'appareil illustré à la figure 1,
- les figures 7b et 7c illustrent différents plans en deux dimensions pouvant être présentés à un opérateur par l'intermédiaire de la sonde de l'appareil illustré à la figure 4,
- la figure 8 est une vue schématique d'un dispositif de guidage selon un deuxième mode de réalisation de l'invention,
- la figure 9a est une vue schématique partielle d'une partie d'un dispositif de guidage selon un troisième mode de réalisation de l'invention,
- les figures 9b à 9d illustrent différents plans en deux dimensions pouvant être présentés à un opérateur par l'intermédiaire de la sonde du dispositif illustré à la figure 9a,
- la figure 10 est une vue schématique d'une partie d'un dispositif de guidage selon un quatrième mode de réalisation de l'invention.

### DESCRIPTION DETAILLEE DE L'INVENTION

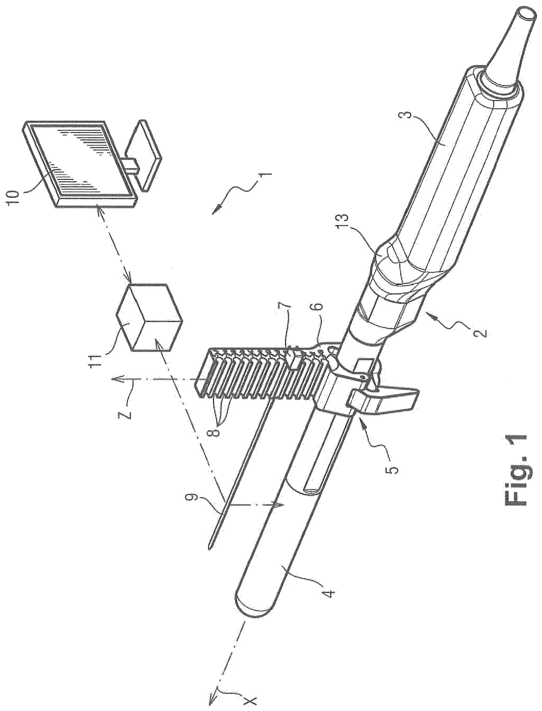

En référence à la figure 1, le dispositif de guidage selon un premier mode de réalisation de l'invention, généralement désigné en 1, est ici mis en oeuvre pour une biopsie d'une prostate d'un patient et ce par une approche transpérinéale.

Cette application n'est bien entendu pas limitative et on pourra utiliser le dispositif de guidage 1 pour d'autres actes médicaux comme une ablation et/ou pour d'autres cibles et par exemple d'autres zones particulières au sein d'autres volumes anatomiques comme un rein, un foie, un utérus etc.

Le dispositif de guidage 1 comporte un appareil 2 de contrôle s'étendant généralement longitudinalement selon un axe X afin de faciliter son insertion dans le rectum du patient.

De préférence, l'appareil 2 comporte un support 3, s'étendant parallèlement à l'axe X, qui est destiné à être saisi au niveau de son extrémité proximale par un opérateur et qui est prolongé à son extrémité distale par un décrochement 13 de l'appareil 2. Ledit décrochement est lui-même prolongé par une sonde 4 d'imagerie médicale de l'appareil pour permettre la génération d'images en trois dimensions de la prostate et de son environnement, sonde s'étendant selon l'axe X.

De la sorte, l'appareil 2 est plus aisément manipulable par l'opérateur du fait du décrochement 13.

La sonde 4 est ici une sonde échographique qui est donc destinée à être introduire dans le rectum du patient.

Ainsi, dans le cas présent, le dispositif de guidage 1 selon le mode de réalisation particulier de l'invention est mis en oeuvre pour la biopsie de la prostate du patient, par une approche transpérinéale, et sous imagerie échographique endorectale.

Cette application n'est bien entendu pas limitative et on pourra utiliser le dispositif de guidage 1 avec d'autres types d'imageries i.e. d'autres types de sondes. On pourra ainsi utiliser une sonde générant directement des images ou bien une sonde permettant de générer des images à partir des informations qu'elle transmet. La sonde pourra ainsi être une sonde de type échographique. De la même façon, la sonde peut générer des images deux dimensions (le dispositif de guidage 1 recréant alors des images trois dimensions à partir des différentes images deux dimensions fournies par la sonde) ou la sonde peut générer directement des images trois dimensions. La sonde pourra être motorisée ou non motorisée. Un balayage effectué par la sonde pourra être assuré via une rotation de la sonde ou via une sollicitation de différents capteurs agencés sur la sonde. Par exemple la sonde pourra comporter des rangées de capteurs piézoélectriques s'étendant toutes parallèlement les unes aux autres, les rangées étant activées les uns après les autres pour obtenir des images deux dimensions selon des orientations différentes.

Ainsi ici la sonde 4 est une sonde fournissant directement des images en trois dimensions ou une sonde 4 fournissant des images en deux dimensions dont le traitement permet de créer une image en trois dimensions.

La sonde 4 est ici choisie parmi les sondes suivantes :
- une sonde fournissant des images en deux dimensions et motorisée afin de pouvoir générer des images en deux dimensions selon des orientations différentes,
- une sonde fournissant des images en deux dimensions et comprenant des capteurs agencés à des endroits différents de la sonde afin de pouvoir générer des images en deux dimensions selon des orientations différentes, mais sans que la sonde ne bouge, par actionnement de capteurs différents de la sonde,
- une sonde fournissant des images en trois dimensions par synthèse d'images en deux dimensions acquises par son ou ses capteurs (soit par motorisation de la sonde soit par sollicitation de capteurs agencés à des endroits différents de la sonde comme proposé ci-dessus),
- une sonde fournissant des images en trois dimensions directement par acquisition de flux de données trois dimensions.

De préférence, la sonde 4 fournit à intervalles réguliers des nouvelles images de façon automatique ce qui permet un rafraîchissement automatique des informations proposées à l'opérateur.

Les images sont rafraîchies également soit lorsque la sonde 4 a effectué un nouveau balayage complet soit au fur et à mesure d'un nouveau balayage de la sonde 4 (permettant par exemple un rafraîchissement en « tranches » de l'image volumique).

La sonde 4 est ainsi une sonde dite « quatre dimensions » ou « 4D » du fait qu'elle permette d'acquérir un flux de données volumique rafraîchi dans le temps (le temps formant alors la quatrième dimension).

Dans tous les cas, si un balayage est nécessaire pour pouvoir générer au final une image en trois dimensions, le balayage effectué par la sonde 4 est soit motorisé (la sonde comporte un moteur), soit électronique (sollicitation de capteurs différents) mais n'est en aucun cas manuel (c'est-à-dire que c'est à l'opérateur de déplacer manuellement la sonde pour pouvoir acquérir des nouvelles images en deux dimensions permettant l'établissement d'une image en trois dimensions).

De façon particulière, la sonde 4 est ici une sonde échographique motorisée. Ladite sonde 4 comporte ainsi une barrette échographique permettant de générer des images en deux dimensions et un moteur agencé dans la sonde 4 de sorte qu'une rotation du moteur entraine une rotation de la barrette échographique autour d'un axe longitudinal Y de la sonde 4. La sonde est ici agencée dans le support de contrôle de sorte que l'axe de rotation de la barrette échographique soit confondu avec l'axe X.

En service, le moteur entraîne en rotation la barrette échographique sur une plage de rotation centrée autour d'une position de référence de la barrette (qui est aussi celle de la sonde 4) de sorte que la barrette puisse acquérir des images en deux dimensions sur cette plage d'angle donnée, images à partir desquels on reconstruit une image volumique correspondante. La sonde 4 est en outre conformée de sorte que le moteur puisse amener la barrette dans une nouvelle position de référence et/ou de sorte que la plage de rotation autour d'une position de référence donnée puisse être modifiée. La sonde 4 est ici conformée de sorte que la barrette puisse effectuer un balayage continu de + ou - X degrés autour de sa position de référence, X étant compris entre 0 et 90, et de préférence entre 0 et 85, et choisi en service selon l'application visée (recherche d'un point particulier, d'une coupe particulière ...).

Le moteur est typiquement commandé de telle sorte que les images en trois dimensions générées à partir des données transmises par la sonde 4 soient rafraîchies à une fréquence comprise entre 0.1 et 6.5 Hertz, et de préférence entre 0.2 et 6 Hertz, lors d'un balayage de la sonde 4. Ceci permet d'afficher dans le mode de réalisation particulier de l'invention des images volumiques à l'opérateur en « 4D » facilitant ainsi son travail.

Le dispositif de guidage 1 comporte par ailleurs un instrument médical. L'instrument comporte par exemple une aiguille 9 à biopsie qui est montée dans un porte-aiguille 5 solidaire de l'appareil de contrôle 2 et ici coulissante dans ledit porte-aiguille 5. De la sorte, l'aiguille 9 est portée par l'appareil de contrôle 2 via le porte-aiguille 5 dudit appareil de contrôle 2. Le porte-aiguille 5 est ici agencé sur l'appareil de contrôle 2 en aval du décrochement 13 (selon un sens allant du support 3 à la sonde 4).

De manière particulière, le porte-aiguille 5 comporte un plateau 6 s'étendant selon un axe Z sensiblement perpendiculairement à l'axe X et comprenant une pluralité de rainures 8 parallèles les unes aux autres (dont une seule est référencée ici) et à l'axe X. Le porte-aiguille 5 comporte également un clou 7 pouvant être déplacé d'une rainure 8 à une autre pour former avec la rainure 8 en regard, un orifice à travers lequel l'aiguille 9 peut être insérée.

Le clou 7 facilite la manipulation de l'aiguille 9 notamment en limitant les mouvements parasites de l'aiguille 9.

L'aiguille 9 peut ainsi être insérée à une hauteur différente dans le porte-aiguille 5 ce qui permet de définir la hauteur à laquelle l'aiguille 9 vient s'enfoncer dans le périnée.

Le clou 7 est ici d'une longueur inférieure à celle d'une rainure et est agencé au niveau de l'extrémité proximale de la rainure associée. Alternativement, le clou 7 est de même longueur que la rainure.

De préférence le clou 7 est dans une couleur différente de celle du plateau 6 afin de faciliter un repérage de l'orifice à travers lequel insérer l'aiguille 9.

Préférentiellement le plateau 6 lui-même comporte des repères graphiques (graduations, caractères alphanumériques ...) pour identifier les différentes rainures 8 du plateau 6 i.e. les différents niveaux du plateau 6.

Ainsi avec un même outil (appareil 2 associé à l'instrument 9) et dans un même mouvement, l'opérateur peut positionner la sonde 4 à proximité de la prostate, en introduisant au moins l'extrémité distale de la sonde 4 dans le rectum du patient, tout en positionnant le porte-aiguille 5 et l'aiguille 9 associée contre le périnée en prévision de la biopsie à venir.

Ceci simplifie donc le pilotage du dispositif de guidage 1.

Par ailleurs, le dispositif de guidage 1 comprenant également un écran 10 et une unité de commande 11 du dispositif de guidage 1.

Le dispositif de guidage 1 comporte également une interface homme-machine (non visible ici) comme par exemple une souris et/ou un clavier et/ou une dalle tactile (éventuellement confondue avec l'écran 10 sur lequel les images sont affichées) permettant à l'opérateur d'interagir avec le dispositif de guidage 1.

Ainsi l'unité de commande 11 est reliée à la sonde 4 et à l'écran 10 pour pouvoir générer ici à l'écran 10 au moins une image en trois dimensions à partir de données émises par la sonde 4 (en travaillant ici en 4D). Dans le cas présent, c'est donc l'unité de commande 11 qui traite les images deux dimensions émises pour créer l'image volumique correspondante projetée sur l'écran 10. C'est également l'unité de commande 11 qui contrôle ici la sonde 4 et notamment la rotation de la barrette.

L'unité de commande 11 est en outre agencée pour générer à l'écran 10 également au moins une image en deux dimensions à partir des données transmises par la sonde 4 et ce sans bouger le support 3 (par exemple en travaillant à partir de l'image en trois dimensions générale générée à partir des données deux dimensions de la sonde 4, ou encore directement à partir des données deux dimensions de la sonde 4 ou encore en déplaçant volontairement la sonde 4 via le moteur). Comme indiqué ci-dessus, cette image est donc rafraichie soit lorsque la sonde 4 a effectué un nouveau balayage total soit lorsque la sonde 4 a effectué un nouveau balayage partiel.

L'unité de commande 11 est ainsi apte à générer à partir des données fournies par la sonde 4, sans bouger le support 3, et avec un rafraîchissement automatique, à la fois une image en trois dimensions mais également :
- une image centrale 2D, image définie par un plan de coupe centrale dans l'image en trois dimensions comprenant l'axe X et correspondant sensiblement à l'image 2D acquise par la sonde 4 dans sa position de référence, et/ou
- une image 2D définie par un plan parallèle à celui de l'image centrale, et/ou
- une image 2D définie par un plan incliné ou perpendiculaire à celui de l'image centrale, par exemple une image 2D dite « orthogonale » et normale à l'axe X, et/ou
- une image 2D définie par une surface courbe,
- etc....

De préférence, l'unité de commande 11 est configurée de sorte à symboliser sur l'image en trois dimensions, le ou les plans, surfaces courbes définissant les autres images 2D également présentes à l'écran 10. Par exemple l'unité de commande 11 affiche sur l'écran 10 le plan de l'image centrale 2D et simultanément l'image en trois dimensions dont est issu ladite image centrale avec une représentation (par exemple par une couleur différente) du plan à partir duquel l'image centrale a été générée.

Ceci facilite l'interprétation de l'image centrale par l'opérateur.

Selon un mode de réalisation particulier, l'unité de commande comporte une mémoire pour l'enregistrement de différentes données et en particulier des différentes images affichées à l'écran 10. Ces enregistrements peuvent être automatiques et/ou commandés par l'opérateur via l'interface homme/machine.

Les données enregistrées dans la mémoire sont par exemple exploitées par le dispositif lui-même ou par un organe externe. Par exemple, bien que non exclusivement, les données enregistrées sont utilisées pour modifier une cartographie des biopsies à prévoir. Selon un mode de réalisation préféré, l'unité de commande 11 est conformée pour optimiser automatiquement la qualité des images en deux dimensions affichées à l'écran 10. L'optimisation peut se baser par exemple sur l'instrument et/ou la cible visée et/ou potentiellement une zone particulière de l'image en trois dimensions saisie par l'opérateur.

Typiquement, l'unité de commande 11 adapte au moins l'un des paramètres de la sonde 4 (et/ou l'un des paramètres de l'image (focus, fréquence des ultrasons, plage dynamique, gain, luminosité, contraste ... par exemple) pour optimiser la qualité des images projetées à l'écran 10.

L'unité de commande peut être agencée pour optimiser individuellement la qualité de chaque image et/ou pour optimiser, de manière globale, différentes images.

Afin de faciliter le guidage de l'instrument, l'unité de commande 11 est par ailleurs configurée de sorte à projeter sur l'écran 10 au moins une image en deux dimensions comprenant ici au moins un point de l'instrument, image qui est donc régulièrement et automatiquement rafraîchie.

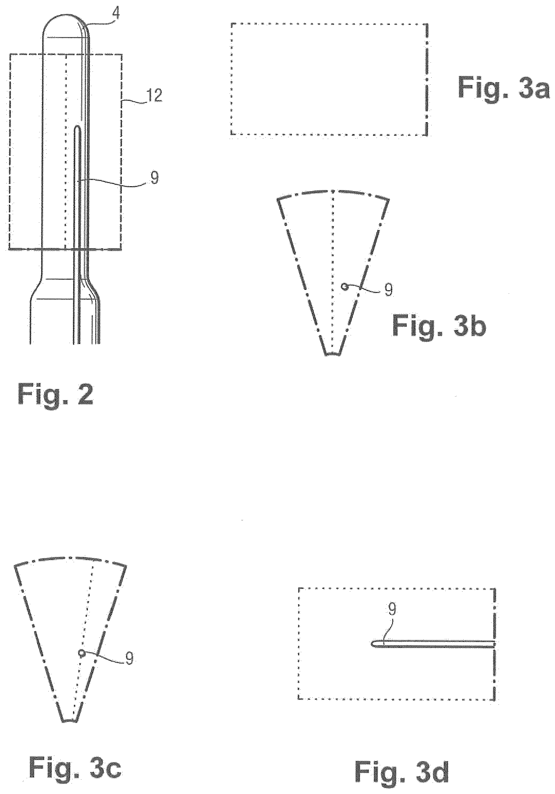

En référence aux figures 2 et 3a à 3d, et selon une mise en oeuvre particulière, le point est le point d'insertion de l'aiguille 9 dans le volume anatomique défini par l'image en trois dimensions. Ledit point d'insertion se trouve généralement (mais pas exclusivement) dans un volume interne au patient situé entre le périnée et la prostate, volume interne qui est donc dans l'environnement immédiat de la prostate. Le point d'insertion de l'aiguille 9 est ainsi le point d'introduction de l'aiguille 9 dans le volume anatomique (i.e. un point d'intersection entre l'aiguille 9 et une zone proximale du volume anatomique).

Avant l'insertion de l'aiguille, l'unité de commande 11 contrôle la sonde 4 afin que sa position de référence soit centrée relativement au porte-aiguille 5. De la sorte, le point d'insertion de l'aiguille 9 sera présent sur l'image centrale 2D projetée sur l'écran 10 (défini par un plan de coupe passant alors par les axes X et Z). L'unité de commande 11 ordonne alors le balayage de la sonde 4 autour de la position de référence et de l'axe X. L'espace ainsi parcouru par la sonde 4 est ici symbolisé en tirets sur la figure 2 et référencé 12. L'opérateur insère ensuite l'aiguille 9 dans le périnée. L'aiguille 9 apparait alors dans l'image volumique en trois dimensions.

Si l'aiguille 9 s'étend bien dans le plan du plateau 6 du porte-aiguille 5, l'unité de commande 11 affiche alors sur l'écran 10 l'image centrale 2D qui va ainsi comprendre le point d'insertion de l'aiguille 9. A cet effet, l'unité de commande 11 récupère directement ladite image centrale 2D de la sonde 4. On affiche ainsi à l'écran 10 une nouvelle image 2D fournissant une information importante à l'utilisateur.

Toutefois, si l'aiguille 9 s'est finalement décalée vis-à-vis dudit plan du plateau 6, l'image centrale ne montre donc ni le point d'insertion de l'aiguille 9 ni même l'aiguille 9 (voir la figure 3a représentant l'image centrale).

Dans ce cas, de manière automatique ou suite à une demande de l'opérateur via l'interface homme-machine, l'unité de commande 11 génère à partir de l'image volumique une image proximale, encore appelée orthogonale, 2D définie par un plan de coupe de l'image volumique qui a pour normal l'axe X et qui passe par une frontière du volume anatomique défini par l'image en trois dimensions.

Cette image proximale 2D est optionnellement affichée sur l'écran 10 pour l'opérateur. Comme illustrée à la figure 3b (représentant l'image proximale), cette image proximale 2D comporte effectivement le point d'insertion.

De préférence, de manière automatique ou sur demande de l'opérateur, l'unité de commande 11 génère sur l'écran 10 au moins une nouvelle image 2D comprenant le point d'insertion. Cette nouvelle image 2D est par exemple :
- une image 2D parallèle à l'image centrale 2D et incluant donc le point d'insertion (l'unité de commande 11 extrait ladite image de l'image volumique),
- une image 2D oblique à l'image centrale 2D et comprenant l'axe X et le point d'insertion (l'unité de commande 11 extrait ladite image de l'image volumique),
- une nouvelle image centrale 2D incluant le point d'insertion comme illustré à la figure 3d qui représente ladite nouvelle image (l'unité de commande 11 requérant alors une rotation de la sonde 4 afin d'aligner la position de référence de la sonde 4 avec l'aiguille 9 comme illustré à la figure 3c; l'unité de commande 11 identifiant l'aiguille 9 par traitement d'images et provoquant un déplacement de la sonde 4 à l'aide de la détermination de la position de l'aiguille 9 vis-à-vis de la sonde 4, image qui peut donc être directement générée par la sonde 4 sans passer par l'image volumique). On retient en variante que si la sonde 4 n'est pas motorisée mais autorise un balayage électronique, on peut assurer la génération de cette nouvelle image centrale par activation d'autres capteurs de la sonde (balayage électronique et non plus motorisé).

On affiche ainsi à l'écran 10 au moins une image comprenant le point d'insertion de l'aiguille 9 ce qui permet de faciliter le guidage de l'aiguille 9. Bien entendu plusieurs images précitées peuvent être affichées simultanément à l'écran 10.

Ceci permet de visualiser une autre partie du corps du patient et de l'instrument sans avoir à bouger le support 3.

De façon avantageuse, ces différentes images sont ici rafraîchies de manière automatique.

Typiquement, lors du balayage de la sonde 4 lors de la recherche du point d'insertion, la barrette effectue un balayage continu de + ou - A degrés autour de sa position de référence, A étant compris entre 5 et 15, et est de préférence sensiblement de 10. La vitesse de rotation est alors définie autour de sensiblement 60 degrés par seconde.

Les images affichées sont alors rafraîchies automatiquement à une fréquence de sensiblement 6 Hertz ce qui permet de travailler en 4D. On note alors que les images deux dimensions et l'image volumique sont rafraîchies à la même fréquence l'image volumique étant rafraîchie dans les deux sens de rotation du moteur).

De préférence, indépendamment des images affichées, une fois le point d'insertion identifié, l'unité de commande 11 amène la sonde 4 de sorte à aligner la position de référence de la sonde 4 avec le point d'insertion de l'aiguille 9 et réduit l'amplitude du balayage de la sonde 4 afin de recentrer le balayage de la sonde 4 au niveau de la zone de travail sur la prostate, assurant ainsi un rafraîchissement plus régulier des différentes images.

Selon un mode de réalisation particulier, si l'opérateur n'a plus besoin d'un rafraîchissement de l'image volumique, le balayage de la sonde 4 est temporairement arrêté. Dans ce cas, la sonde 4 n'acquiert plus que l'image centrale 2D (et ce de façon automatique sans avoir à passer par l'image en trois dimensions) mais à une fréquence plus importante assurant un rafraîchissement plus régulier de l'image. Ladite fréquence est supérieure à 15 Hertz. Typiquement cette image est rafraîchie à une fréquence de sensiblement 20 Hertz. On travaille alors là encore par rafraîchissement automatique mais seulement au niveau de l'image centrale 2D.

De façon particulière, on enregistre dans la mémoire, un ou des paramètres et/ou un ou une image afin que le point d'insertion et/ou l'orientation de l'aiguille 9 puissent être enregistrées. Ces données sont ensuite exploitées par exemple en vue d'afficher un positionnement prenant en compte ces différentes données dans la cartographie des biopsies ou du traitement.

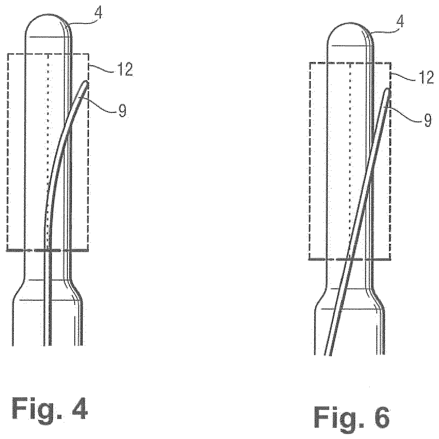

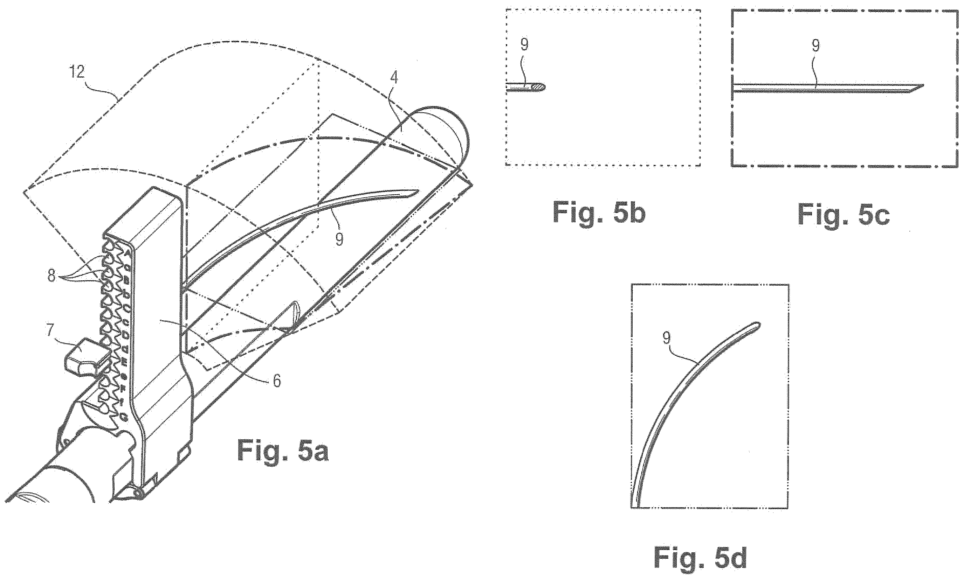

En référence aux figures 4 et 5a à 5d, et selon une mise en oeuvre particulière, le point est la partie utile de l'instrument (soit la partie de l'instrument destinée à agir sur la prostate). Dans le cas présent, la partie utile est l'extrémité libre de l'aiguille 9 (c'est-à-dire celle destinée à effectuer la biopsie).

L'unité de commande 11 contrôle la sonde 4 afin que sa position de référence soit centrée relativement au porte-aiguille 5 (ou comme indiqué en option dans le premier mode de réalisation afin que sa position de référence soit centrée relativement au point d'insertion de l'aiguille 9). De la sorte, l'extrémité libre supposée de l'aiguille 9 dans la prostate sera présente sur l'image centrale 2D projetée sur l'écran 10. L'unité de commande 11 ordonne alors le balayage de la sonde 4 autour de la position de référence et de l'axe X. L'espace 12 ainsi parcouru par la sonde 4 est ici symbolisé en tirets sur la figure 4.

Si l'aiguille 9 s'étend bien jusque dans la prostate dans le plan de l'image centrale 2D, l'unité de commande 11 affiche alors sur l'écran 10 ladite image centrale 2D qui va ainsi comprendre l'extrémité libre de l'aiguille 9. A cet effet, l'unité de commande 11 récupère directement ladite image centrale 2D de la sonde 4. On affiche ainsi à l'écran 10 une nouvelle image 2D fournissant une information importante à l'utilisateur.

Toutefois, si l'aiguille 9 s'est finalement décalée vis-à-vis dudit plan, l'image centrale ne montre donc pas l'extrémité libre de l'aiguille 9 comme visible à la figure 5b. En particulier l'aiguille 9 est aisément déformable de par sa forme. Les déformations de l'aiguille 9 peuvent être indésirables, mais également souhaitées et contrôlées. En effet, de nouvelles techniques permettent d'exploiter la déformation des aiguilles pour accéder à des zones inaccessibles autrement, en contournant des obstacles. Néanmoins dans tous les cas, la déformation de l'aiguille 9 entraîne une non-visualisation de l'extrémité libre de l'aiguille 9 sur l'image centrale.

Dans ce cas, de manière automatique ou suite à une demande de l'opérateur via l'interface homme-machine, l'unité de commande 11 génère sur l'écran 10 au moins une nouvelle image 2D comprenant l'extrémité libre de l'aiguille 9. Cette nouvelle image 2D est par exemple :
- une image 2D oblique, telle qu'illustrée à la figure 5d, définie par un plan de coupe oblique ou orthogonal au plan définissant l'image centrale, ledit plan comprenant l'extrémité libre de l'aiguille 9 (l'unité de commande extrait ladite image 2D de l'image volumique) et étant de préférence parallèle à l'axe X,
- une image 2D oblique, définie par la projection de l'aiguille sur un plan de coupe oblique ou orthogonal au plan définissant l'image centrale, ledit plan comprenant l'extrémité libre de l'aiguille 9 (l'unité de commande extrait ladite image 2D de l'image volumique) et étant de préférence parallèle à l'axe X,
- une image 2D, telle qu'illustrée à la figure 5c, issue d'une surface courbe qui suit la déformation de l'aiguille 9 (l'unité de commande 11 extrayant ladite image de l'image volumique par exemple par détermination de la surface courbe suivant la déformation de l'aiguille 9 et projection de ladite surface courbe sur un plan définissant alors l'image),
- une image 2D issue de la réduction de l'image volumique générale à un plan (par exemple l'unité de commande 11 calcule une moyenne, une somme ou un maximum d'amplitude autour d'un axe donné suivant la position de l'aiguille 9).

On affiche ainsi à l'écran 10 au moins une image comprenant l'extrémité libre de l'aiguille 9 ce qui permet de faciliter le guidage de l'aiguille 9. Bien entendu plusieurs images précitées peuvent être affichées simultanément à l'écran 10.

De façon avantageuse, ces différentes images sont ici rafraîchies de manière automatique ce qui permet de bien suivre l'avancée de l'aiguille 9 dans le patient et simplifie en conséquence le pilotage de l'aiguille 9. En particulier, l'opérateur peut ainsi suivre l'enfoncement de l'aiguille 9 dans la prostate de manière très fine.

Par ailleurs ceci permet de visualiser une autre partie du corps du patient et de l'instrument sans avoir à bouger le support 3. Avantageusement, lesdites images permettent ici de visualiser l'intégralité de l'aiguille 9 insérée dans le patient ce qui facilite encore davantage le pilotage de l'instrument. En particulier, le point d'insertion de l'aiguille 9 est également visualisable ici.

On peut ainsi visualiser toute la courbure de l'aiguille 9.

De préférence, l'unité de commande 11 enregistre dans la mémoire la courbure de l'aiguille 9 (soit l'image, soit des points de coordonnées identifiant la courbure de type point d'insertion et extrémité libre de l'aiguille 9 avec éventuellement des points intermédiaires ...) . Ceci peut être utilisé par la suite pour être intégré à la cartographie des biopsies.

En option, indépendamment des images affichées, une fois l'extrémité libre identifiée, l'unité de commande 11 amène la sonde 4 de sorte à aligner la position de référence de la sonde 4 avec l'extrémité libre de l'aiguille 9 et réduit l'amplitude du balayage de la sonde 4 afin de recentrer le balayage de la sonde 4 au niveau de la zone de travail sur la prostate, assurant ainsi un rafraîchissement plus régulier des différentes images.

Selon un mode de réalisation particulier, si l'opérateur n'a plus besoin d'un rafraîchissement de l'image volumique, le balayage de la sonde 4 est temporairement arrêté. Dans ce cas, la sonde 4 n'acquiert plus que l'image centrale 2D (et ce de façon automatique sans avoir à passer par l'image en trois dimensions) mais à une fréquence plus importante assurant un rafraîchissement plus régulier de l'image. Ladite fréquence est supérieure à 15 Hertz. Typiquement cette image est rafraîchie à une fréquence de sensiblement 20 Hertz. On travaille alors là encore au niveau de l'image centrale 2D par rafraîchissement automatique.

De façon particulière, on enregistre dans la mémoire, un ou des paramètres et/ou un ou une image afin que le point d'insertion et/ou l'orientation de l'aiguille 9 et/ou sa déformation puissent être enregistrées. Ces données sont ensuite exploitées par exemple en vue d'afficher un positionnement prenant en compte ces différentes données dans la cartographie des biopsies ou du traitement ou encore en vue de sauvegarder et d'indiquer les valeurs réelles par rapport aux valeurs théoriques (i.e. sans déformation de l'aiguille 9). Ceci peut également permettre d'anticiper à l'avenir les déformations à prévoir pour les futurs sites de prélèvements.

Cette mise en oeuvre s'applique également lorsque l'aiguille 9 n'est pas déformée de manière courbe mais de manière oblique comme visible à la figure 6.

Dans ce cas de figure, en complément ou en remplacement, l'unité de commande 11 peut générer sur l'écran 10 au moins une nouvelle image 2D confondue avec le plan de l'aiguille 9 (l'unité de commande 11 extrayant ladite image de l'image volumique).

Avec le dispositif de guidage 1 ainsi décrit, on peut ainsi détecter et suivre l'insertion de l'aiguille 9 vers une cible dans la prostate et ce même en approche transpérinéale. De façon particulière, l'unité de commande 11 peut permettre l'insertion de ladite cible (ou de plusieurs cibles) dans le ou les images affichées. Par exemple la cible est représentée par un élément graphique (un cercle, une sphère, une étoile, un élément alphanumérique) et/ou sera dans une couleur différente du reste de l'image. Ceci facilite le travail de l'opérateur en lui permettant notamment de contrôler la distance aiguille 9 - cible.

De façon particulière, l'unité de commande 11 peut permettre l'insertion d'une trajectoire planifiée (soit la trajectoire que l'aiguille 9 doit suivre pour atteindre la cible) dans le ou les images affichées. Par exemple la trajectoire planifiée est représentée par un trait particulier (par exemple en pointillés) et/ou sera dans une couleur différente du reste de l'image. Ceci facilite le travail de l'opérateur en lui permettant notamment de contrôler la distance aiguille 9 - cible.

Ceci permet de mieux piloter l'aiguille 9 et le dispositif médical 1 de manière générale. En particulier, ceci permet à l'opérateur de mieux contrôler la position et la profondeur avec laquelle il insère l'aiguille 9 dans la prostate.

La cible et/ou la trajectoire planifiée sont par exemple déterminées en amont de la biopsie lors de la planification de la biopsie par exemple ou bien sont définies au début de la biopsie après l'acquisition de premières images du patient et notamment de la prostate.

Les informations transmises par la sonde 4 permettent donc d'explorer la prostate et son environnement immédiat selon différents points de vue sans bouger l'appareil 2, voire sans bouger la position de référence de la sonde 4, en vue d'identifier le point d'insertion, la partie utile, l'angle d'insertion, ainsi que la courbure de l'aiguille 9.

On note que l'affichage de telles images ne serait pas disponible sans le dispositif de guidage 1 qui permet ainsi d'afficher des images de coupes obliques, courbes, orthogonales ...

En outre, les images sont rafraîchies de manière automatique permettant un bon suivi du l'aiguille 9.

Avantageusement, le dispositif de guidage 1 ne nécessite aucun capteur particulier pour la génération des images en deux dimensions comprenant le point d'insertion ou la partie utile de l'instrument.

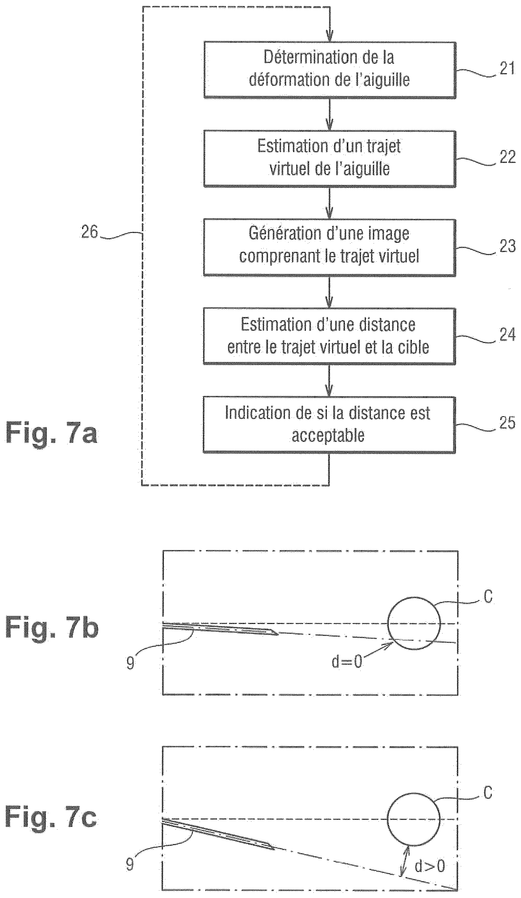

Une autre mise en oeuvre du dispositif va être à présente décrite en référence à la figure 7a.

Comme indiqué précédemment, l'aiguille 9 est introduite dans le corps du patient en s'aidant des images acquises par la sonde 4 en particulier des images en deux dimensions et/ou en trois dimensions.

Si l'aiguille 9 atteint la cible visée dans la prostate (ce qui peut être vérifié par exemple par des images en deux dimensions et/ou en trois dimensions de la prostate), la biopsie est réussie et s'achève ici.

Si au contraire on constate une déformation non souhaitée de l'aiguille 9 sur les images et/ou que l'aiguille dévie de la trajectoire planifiée, il existe un risque que l'aiguille 9 n'atteigne pas la cible.

La détection de la déformation de l'aiguille 9 peut se faire manuellement, par détection visuelle, ou automatiquement par exemple par traitement d'images en comparant la trajectoire planifiée à la trajectoire réelle de l'aiguille 9. Une partie des étapes qui suivent pour déterminer le trajet virtuel de l'aiguille 9 peut ainsi être commune à la détection automatique de la déformation de l'aiguille 9.

Dans ce cas, au cours d'une première étape 21, l'unité de commande 11 détermine la déformation de l'aiguille 9.

Typiquement, l'unité de commande 11 calcule une ligne en trois dimensions représentant l'aiguille 9 déformée selon les étapes suivantes :
a) sélection de différents points de l'aiguille 9 sur l'image volumique et/ou des images deux dimensions extraites de l'image volumique (sélection faite manuellement par l'opérateur ou automatiquement par l'unité de commande par exemple par traitement d'images ; de préférence la sélection se fera à partir d'images deux dimensions), et
b) interpolation de ces différents points via une fonction polynomiale, une courbe de Bézier, une fonction spline (moins connue sous le terme français de fonction cerce) pour calculer la ligne en trois dimensions représentant l'aiguille 9 déformée.

Dans les points sélectionnés à l'étape a, on sélectionne de préférence au moins le point d'insertion de l'aiguille 9 et le point de l'extrémité libre de l'aiguille 9. On sélectionne ici également entre 3 et 8 points supplémentaires sur la longueur de l'aiguille entre ces deux points.

On note que la ligne en trois dimensions représentant l'aiguille 9 déformée comporte au moins un point de l'instrument et dans le cas présent au moins deux points (le point d'insertion et le point de l'extrémité libre).

De préférence encore, l'unité de commande 11 enregistre dans la mémoire la courbure de l'aiguille 9 (soit l'image, soit des points de coordonnées identifiant la courbure de type point d'insertion et extrémité libre de l'aiguille 9 avec éventuellement des points intermédiaires, soit la ligne en trois dimensions, soit la ligne en deux dimensions ...) . Ceci peut être utilisé par la suite pour un affichage dans la cartographie des biopsies.

Cette mise en oeuvre s'avère relativement avantageusement lorsque l'aiguille 9 n'est pas déformée de manière simple mais de manière complexe comme par exemple de sorte à former une sinusoïde.

On note en outre que cela se fait sans avoir à bouger le support 3.

Au cours d'une deuxième étape 22, une fois que l'unité de commande 11 a déterminé la déformation de l'aiguille 9, l'unité de commande 11 estime un trajet virtuel de l'aiguille 9 si on prolongeait son insertion jusqu'à la cible selon la trajectoire actuelle. En effet, grâce à la ligne en trois dimensions, l'unité de commande 11 connaît la position, l'orientation ainsi que la déformation de l'aiguille entre les points indiqués. L'unité de commande 11 peut facilement en déduire comment cette ligne en trois dimensions continue au-delà du point de l'extrémité libre de l'aiguille 9. Selon un mode de réalisation particulier, l'unité de commande 11 détermine la tangente à ce point et en déduit une prolongation rectiligne de la ligne en trois dimensions. Ceci peut par exemple se faire en définissant une droite perpendiculaire à la tangente et passant par au moins deux points de la ligne précédemment calculée. Lesdits points sont de préférence les points les plus proches de la partie utile de l'aiguille 9.

De la sorte, le trajet virtuel de l'aiguille 9 est déterminé.

L'unité de commande travaille ici dans le repère de la sonde.

Au cours d'une troisième étape 23, l'unité de commande 11 génère à l'écran :
- une image en trois dimensions comprenant ledit trajet virtuel en plus de l'aiguille 9 et/ou de la ligne en trois dimensions (qui pourra ainsi se superposer à l'aiguille 9), et/ou
- une image en deux dimensions définie par un plan coronal (soit un plan orthogonal à celui définissant l'image centrale 2D et comprenant l'axe X) dans l'image en trois dimensions, l'image en deux dimensions comprenant la projection du trajet virtuel en plus de la projection de l'aiguille et/ou de la ligne en trois dimensions symbolisant l'aiguille 9 sur ledit plan coronal, projection définissant ainsi une ligne courbe en deux dimensions, et/ou
- une image définie par un plan passant par la ligne en trois dimensions et/ou par un plan passant par le trajet virtuel, image de préférence affichée avec au moins une image en deux dimensions à côté pour permettre de faciliter la compréhension de ladite image (typiquement on commence par définir une surface en trois dimensions passant par la ligne en trois dimensions et/ou le trajet virtuel, surface qui est définie par des lignes toutes parallèles à l'axe X, Y ou Z et de préférence parallèles à l'axe Z, puis on aplatit cette surface pour obtenir une image en deux dimensions. Par exemple l'image est définie par un plan passant par la ligne en trois dimensions puis passant par le trajet virtuel ce qui permet de générer une image contenant à la fois l'instrument et le trajet virtuel.

Par exemple le trajet virtuel est représenté par un trait particulier (par exemple en pointillés) et/ou sera dans une couleur différente du reste de l'image. Ceci facilite le travail de l'opérateur en lui permettant notamment de contrôler la distance aiguille 9 - cible.

Bien entendu la ou les images affichées peuvent comporter à la fois le trajet virtuel et à la fois la trajectoire planifiée.

Ceci permettra à l'opérateur de mieux se représenter la situation.

Au cours d'une quatrième étape 24, l'unité de commande 11 déduit au moins une distance entre le trajet virtuel et la cible visée.

De préférence, l'unité de commande 11 déduit une distance minimale entre le trajet virtuel et la cible visée.

Par exemple, l'unité de commande 11 détermine une distance euclidienne entre chaque point du trajet virtuel et chaque point de la surface de la cible et en déduit la distance la plus faible.

En variante, l'unité de commande 11 détermine une distance euclidienne entre chaque point du trajet virtuel et le centre de la cible et en déduit la distance la plus faible.

Au cours d'une cinquième étape 25, en fonction de la distance calculée, l'unité de commande 11 indique si cette distance est acceptable.

Optionnellement, l'unité de commande 11 indique si cette distance est nulle, en d'autres termes si l'aiguille 9 attendra bien la cible visée malgré sa déformation.

La figure 7b illustre un cas dans lequel la distance est nulle et la figure 7c un cas dans lequel la distance n'est pas nulle indiquant que l'aiguille 9 n'atteindra pas la cible C (la ligne en tirets représentant la trajectoire planifiée et la ligne en pointillés le trajet virtuel).

Par exemple, l'unité de commande 11 indique que cette distance est acceptable à l'opérateur via l'écran 10 en affichant un message d'avertissement de type information chiffrée, information binaire de type oui/non, information colorée (par exemple rouge/vert) ...

Si la distance est acceptable, l'opérateur peut donc continuer à poursuivre son mouvement. Sinon l'opérateur retire l'aiguille 9 et recommence son geste afin d'atteindre la cible visée ou ajuste la trajectoire de l'aiguille 9 s'il le peut.

De préférence, travaillant ici en 4D, l'unité de commande 11 est configurée pour afficher à l'écran 10 le message d'avertissement précité de manière qu'il soit mis à jour automatiquement en même temps que les images (ce qui est schématisé par les pointillées à la figure 7). On note ainsi qu'ici le trajet virtuel est mis à jour automatiquement que ce soit en termes de calcul ou d'affichage à l'écran 10. De la même façon, la distance entre ledit trajet virtuel et la cible est mise à jour automatiquement.

Ainsi l'opérateur peut poursuivre son geste tant que le message d'avertissement n'indique pas que la distance estimée n'est plus acceptable et qu'il y a un risque que la cible ne soit pas atteinte (passage par exemple d'un indicateur coloré du vert au rouge).

Une autre mise en oeuvre du dispositif va être à présente décrite.

Dans cette autre mise en oeuvre, le dispositif 1 comporte au niveau de son interface homme/machine au moins un bouton de contrôle de la sonde 4 par l'opérateur.

Ledit bouton de contrôle est un bouton de l'angle de rotation du moteur de la sonde 4 ce qui permet à l'opérateur de pouvoir commander à la sonde 4 de se déplacer à une nouvelle position de référence. Le bouton de contrôle est par exemple un curseur de défilement (physique ou virtuel selon la forme de l'interface homme/machine) .

L'interface homme/machine comporte en outre ici au moins un bouton de contrôle des images extraites de l'image volumique. L'interface homme/machine comporte ici un premier bouton de contrôle pour contrôler l'angle relatif entre une image particulière en deux dimensions affichées à l'écran et l'image centrale en deux dimensions correspondant à la position de référence de la sonde 4. De façon particulière, l'interface homme/machine comporte ici un deuxième bouton de contrôle pour contrôler la courbure de l'image particulière (ledit bouton de contrôle permettant ainsi d'augmenter et de diminuer un rayon de courbure de ladite image qui est plane lorsque le rayon de courbure est égal à zéro et courbe de courbure constante lorsque le rayon de courbure n'est pas égal à zéro). Les deux boutons de contrôle sont par exemple des curseurs de défilement (physique ou virtuel selon la forme de l'interface homme/machine). A l'aide de ces deux boutons de contrôle, l'opérateur peut ainsi afficher à l'écran les images qui l'intéresse sans avoir à bouger l'appareil 2, les commandes qu'il effectue via les deux boutons permettant de commander un traitement des images volumiques acquises par la sonde 4.

Typiquement l'opérateur peut ainsi parcourir l'image volumique par succession d'images deux dimensions afin de pouvoir trouver une image deux dimensions qui lui convient. L'opérateur peut également provoquer la rotation de la sonde afin d'obtenir de nouvelles images en trois dimensions plus intéressantes.

De façon particulière, l'opérateur peut également (en cliquant sur l'écran 10 ou en appuyant sur l'écran 10 si celui-ci est tactile) sélectionner des points particuliers de l'image afin que l'unité de commande enregistre les cordonnées desdits points.

Typiquement, si une image en deux dimensions permet de visualiser une déformation de l'instrument, l'opérateur peut sélectionner un ou plusieurs points de l'instrument permettant à l'unité de commande 11 d'enregistrer lesdits points représentatifs de la déformation de l'instrument et/ou pour créer une ligne en trois dimensions représentant l'aiguille 9 déformée.

Par exemple, à l'aide des boutons de commande l'opérateur provoque l'affichage à l'écran de différentes coupes en deux dimensions jusqu'à identifier l'aiguille 9 et sa déformation. L'opérateur peut ainsi choisir une image en deux dimensions comprenant l'extrémité libre de l'aiguille 9. Puis l'opérateur enfonce l'aiguille 9 à la profondeur voulue en s'aidant de ladite image pour guider l'aiguille 9. L'opérateur peut alors sélectionner l'extrémité libre de l'aiguille 9 et le point d'insertion de l'aiguille 9 sur les différentes images en deux dimensions (et éventuellement d'autres points). L'unité de commande 11 enregistre alors les différents points sélectionnés pour une éventuelle utilisation dans la cartographie des biopsies.

Selon un autre exemple, l'opérateur commande la rotation de la sonde pour sélectionner sur les images générées à l'écran le point d'insertion, l'extrémité libre de l'aiguille et éventuellement plusieurs autres points intermédiaires de l'aiguille. L'unité de commande 11 génère alors une ligne en trois dimensions représentant l'aiguille 9 déformée et génère à l'écran 10 une ou des images à partir de ladite ligne en trois dimensions comme indiqué précédemment.

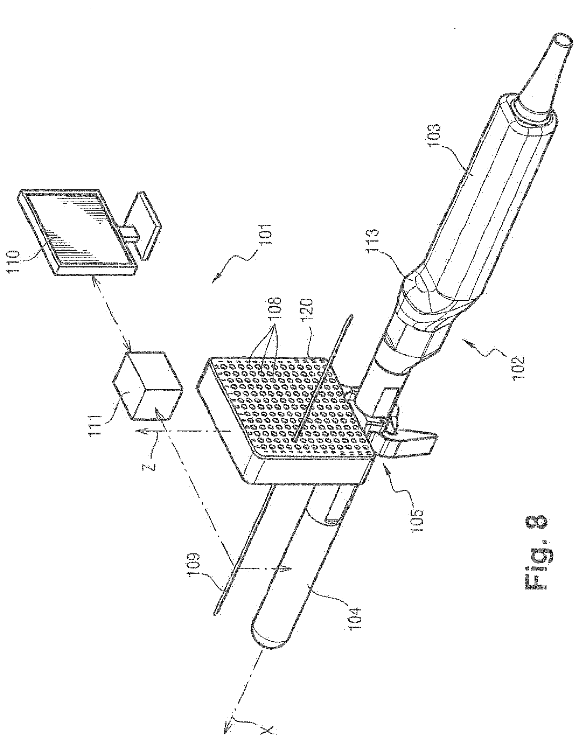

En référence à la figure 8, un dispositif de guidage 100 selon un deuxième mode de réalisation va être à présent décrit. Ce dispositif de guidage est ici identique à celui du premier mode de réalisation mis à part au niveau du porte-aiguille.

Ainsi, à la différence du premier mode de réalisation, le porte-aiguille 105 comporte une grille 120 qui s'étend ici dans un plan sensiblement de normale l'axe X. La grille 120 comporte ainsi des orifices 107, tous parallèles les uns aux autres, et à l'axe X. Les différents orifices 107 sont par ailleurs agencés en colonnes et en lignes sur la grille 120 selon un axe Y, orthogonal à l'axe X, et un axe Z, orthogonal à l'axe X et à l'axe Y.

L'aiguille 109 peut ainsi être insérée à une hauteur différente (selon l'axe Z) dans le porte-aiguille 105 ce qui permet de définir la hauteur à laquelle l'aiguille 109 vient s'enfoncer dans le périnée mais également à une distance différente (selon l'axe Y) dans le porte-aiguille 105 ce qui permet de définir la distance à laquelle l'aiguille 109 vient s'enfoncer dans le périnée.

On peut ainsi guider l'aiguille 109 tout en ayant une liberté selon deux degrés de liberté sur la zone d'insertion de l'aiguille 109 dans le périnée.

Préférentiellement la grille 120 elle-même comporte des repères graphiques (graduations, caractères alphanumériques ...) pour identifier les différentes colonnes d'orifices de la grille 120 et des repères graphiques différents pour identifier les différentes lignes d'orifices de la grille 120.

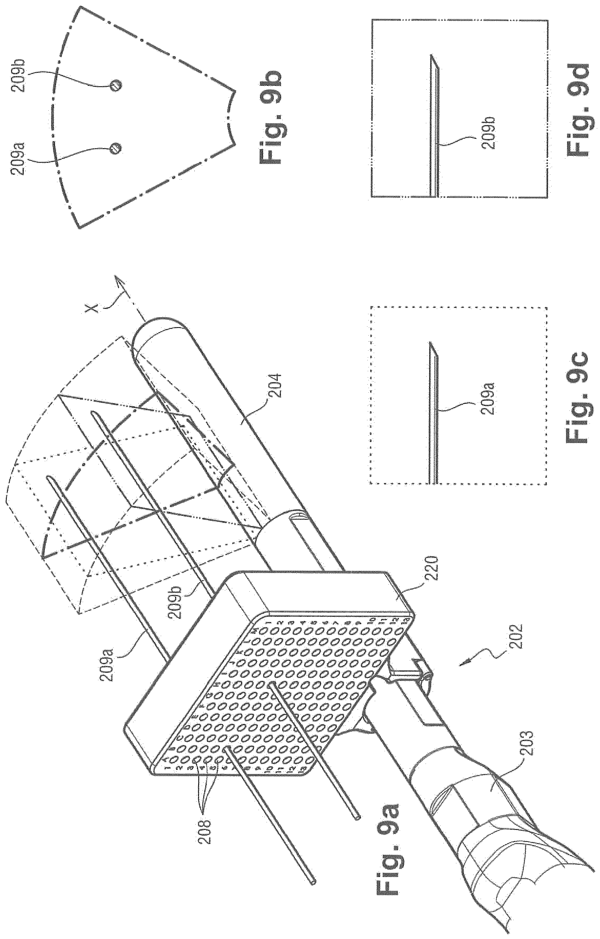

Un troisième mode de réalisation va être à présent décrit en référence aux figures 9a à 9d.

Ce dispositif de guidage est ici identique à celui du premier mode de réalisation mis à part au niveau de l'instrument médical. En effet, alors que dans le premier mode de réalisation, le dispositif comportait un seul instrument, dans le présent mode de réalisation le dispositif comporte deux instruments.

Le premier instrument est ainsi une première aiguille 209a à biopsie et le deuxième instrument est ainsi une deuxième aiguille 209b

Les deux aiguilles sont ici montées dans un porte-aiguille identique à celui du deuxième mode de réalisation de sorte à pouvoir coulisser dans le porte-aiguille. Alternativement, les deux aiguilles seront montées dans le porte-aiguille du premier mode de réalisation.

On peut ainsi guider les deux aiguilles 209a, 209b selon deux degrés de liberté grâce aux différents orifices de la grille 220 et ce de manière indépendante d'une aiguille à une autre.

Ainsi avec un même outil et dans un même mouvement, l'opérateur peut positionner la sonde 204 à proximité de la prostate, en introduisant au moins l'extrémité distale de la sonde 204 dans le rectum du patient, tout en positionnant le porte-aiguille et les aiguilles 209a, 209b associées contre le périnée en prévision de la biopsie à venir.

Comme pour le premier mode de réalisation, l'unité de commande permet à partir des données fournies par la sonde 204 de créer une image volumique en trois dimensions puis de travailler sur ladite image volumique pour en extraire des images en deux dimensions travaillées.

De préférence, on affiche ici à l'écran, simultanément ou alternativement :
- une première image en deux dimensions rafraîchie automatiquement incluant un point du premier instrument 209a soit ici le point d'introduction dans le volume anatomique défini par l'image en trois dimensions (correspondant à la figure 9c),
- une deuxième image en deux dimensions rafraîchie automatiquement incluant un point du deuxième instrument 209b soit le point d'introduction dans le volume anatomique défini par l'image en trois dimensions (correspondant à la figure 9d), et
- une troisième image en deux dimensions rafraîchie automatiquement incluant un point du premier instrument 209a soit le point d'introduction dans le volume anatomique défini par l'image en trois dimensions et un point du deuxième instrument 209b soit le point d'introduction dans le volume anatomique défini par l'image en trois dimensions (correspondant à la figure 9b) .

Par exemple la première image est issue d'un plan de coupe incliné par rapport au plan de coupe centrale, plan de coupe comprenant ainsi l'axe de l'aiguille 209a ; la deuxième image est issue d'un plan de coupe incliné par rapport au plan de coupe centrale, plan de coupe comprenant ainsi l'axe de l'aiguille 209b et la troisième image est issue d'un plan de coupe normal à l'axe X et comprenant ainsi les sections des deux aiguilles 209a et 209b.

Bien entendu d'autres images peuvent être affichées à l'écran. Par exemple, l'unité de commande peut générer trois plans orthogonaux entre eux pour chaque instrument permettant de mieux appréhender la position de chaque instrument dans l'espace. Par exemple, pour chaque instrument, une première image deux dimensions incluant l'axe de l'instrument, une deuxième image deux dimensions normale audit axe et une troisième image orthogonale à la première image et à la deuxième image sont générées par l'unité de commande.

Bien entendu, on peut également afficher les trajectoires planifiées et/ou les trajets virtuels pour l'un ou tous les instruments comme déterminés en référence aux figures 7a à 7c.

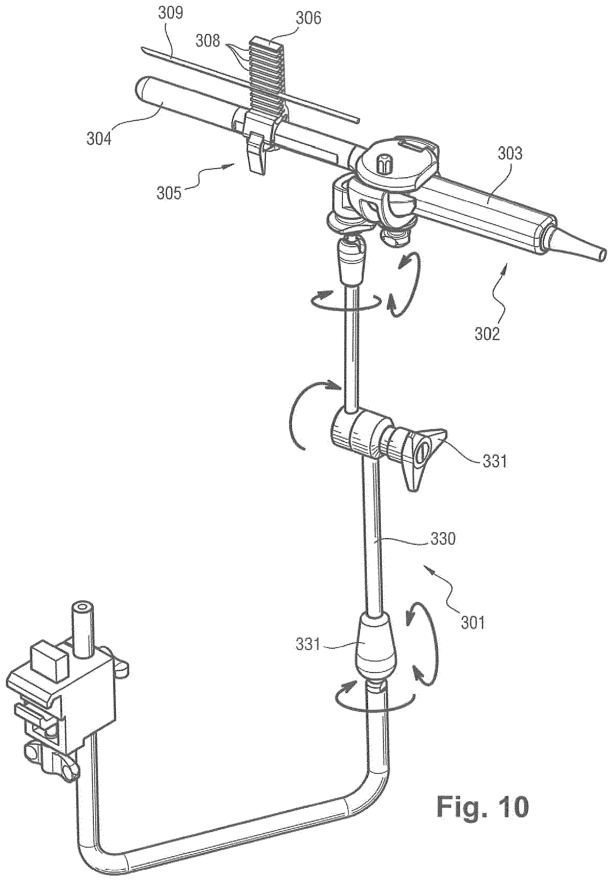

Un quatrième mode de réalisation va être à présent décrit en référence à la figure 10.

Alors que dans le premier mode de réalisation, le dispositif de guidage 1 ne nécessitait aucun dispositif de maintien particulier de l'appareil 2, dans le présent mode de réalisation, le dispositif de guidage 301 comporte un robot ou un bras associé à l'appareil 302.

Le dispositif de guidage 301 comporte ici un bras 330 portant l'appareil 2. Ledit bras 330 est raccordé à son autre extrémité à une structure fixe (non visible ici) .

Le bras 330 comporte ici des moyens pour régler les dimensions du bras 330. Le bras 330 comporte par exemple des molettes 331 permettant de régler la hauteur du bras et l'inclinaison de l'appareil 302 relativement au bras 330.

De préférence, le bras 330 est relié à l'appareil 302 au niveau du décrochement entre le support 303 et la sonde 304 ce qui facilite la manipulation de l'appareil 302.

Par ailleurs, préférentiellement, l'appareil 302 est ici monté de manière amovible sur ledit bras 330 ce qui permet à l'opérateur de reprendre la main à tout moment.

En dehors du bras 330, le dispositif selon le quatrième mode de réalisation est identique à celui du premier mode de réalisation.

Bien entendu l'invention n'est pas limitée aux modes de réalisation décrits et on peut y apporter des variantes sans sortir du cadre de l'invention tel que défini par les revendications.

En particulier, le dispositif pourra comporter un nombre différent d'instruments et/ou comporter des instruments différents de ce qui a été indiqué en fonction des applications visées. En fonction de l'application à laquelle sera destiné le dispositif, l'instrument pourra ainsi changer. L'instrument pourra être longiforme ou non. L'instrument pourra ainsi être un instrument de biopsie, de traitement, un instrument chirurgical, une aiguille, un applicateur, une fibre ... Si le dispositif comporte au moins deux instruments, le dispositif pourra ainsi comporter au moins deux applicateurs de cryothérapie, au moins deux fibres laser ...

Dans le cas où le dispositif comporte plusieurs instruments (applicateurs, aiguilles ...), l'invention sera de préférence bien que non exclusivement agencée pour pouvoir générer à l'écran au moins une image par instrument afin qu'au moins un point de chaque instrument puisse être visible simultanément à l'écran si besoin. L'unité de commande pourra alors extraire des données issues de la sonde, au moins une image deux dimensions associée à chaque instrument. L'unité de commande générera de préférence entre deux à trois plans orthogonaux entre eux pour chaque instrument permettant de mieux appréhender la position de chaque instrument dans l'espace. Par exemple, pour chaque instrument, une première image deux dimensions pourra inclure l'axe de l'instrument, une deuxième image deux dimensions pourra être normale audit axe et une troisième image pourra être orthogonale à la première image et à la deuxième image.

L'unité de commande pourra également générer une image en deux dimensions commune aux deux instruments et incluant ainsi au moins un point de chacun des instruments.

Bien qu'ici l'image en trois dimensions soit projetée à l'écran, le dispositif pourra être agencé pour générer une image en trois dimensions mais n'afficher à l'écran que les images en deux dimensions extraites de ladite image en trois dimensions.

Les images en deux dimensions affichées pourront permettre de visualiser une déformation aussi bien temporaire de l'instrument (comme c'est le cas ici de l'aiguille) ou permanente (si l'instrument est naturellement d'une forme non rectiligne par exemple).

En cas de déformation importante, le dispositif pourra avoir recours à deux images volumiques prises pour des positions de références de la sonde différentes, afin d'obtenir une image en deux dimensions comme une image montrant toute la déformation de l'instrument.

Les différentes mises en oeuvre précitées et modes de réalisation précités pourront être combinés, partiellement ou en totalité.

On pourra ainsi présenter différentes combinaisons d'images affichées à l'écran autres que celles indiquées notamment bien que non exclusivement en fonction des applications visées. Par exemple l'affichage de coupes obliques sera pertinent lors d'une insertion oblique de l'instrument par rapport à l'image centrale.

L'unité de commande pourra insérer dans le ou les images affichées une cible ou des cibles à atteindre pour effectuer un acte médical. Par exemple la cible sera représentée par un élément graphique (un cercle, une sphère, une étoile, un élément alphanumérique) et/ou sera dans une couleur différente du reste de l'image.

Par ailleurs, l'unité de commande sera configurée pour afficher une image comprenant au moins le point de l'instrument soit de manière automatique (par exemple par traitement d'images permettant de détecter l'instrument sur l'image volumique) soit sur demande de l'opérateur (l'opérateur donnant alors l'ordre pour lancer la détection automatique de l'instrument ou sélectionnant à l'écran dans l'image volumique au moins le point de l'écran à viser pour la génération de la nouvelle image).

Comme indiqué dans la description, le dispositif pourra être agencé pour que l'opérateur puisse décider via l'interface homme/ machine quelle(s) image(s) il souhaite voir afficher à l'écran. En particulier, la position et/ou l'orientation et/ou la courbure des plans et surfaces définissant les différentes images seront par exemple des paramètres contrôlables par l'opérateur. De la même façon, l'opérateur pourra optionnellement commander de lui-même une nouvelle position de la sonde en indiquant la position de référence et/ou l'amplitude du balayage (électronique ou motorisé) qu'il souhaite voir appliquer par la sonde.

L'unité de commande pourra être configurée pour détecter automatiquement l'instrument à partir des données fournies par la sonde (par exemple par traitement d'au moins une image volumique générée à partir des données de la sonde). L'unité de commande pourra alors être configurée pour présenter automatiquement au moins un plan comprenant au moins un point de l'instrument (soit le plan le plus adapté au contrôle de l'instrument). L'unité de commande pourra contrôler la sonde pour définir un nouveau balayage de la sonde afin de le réduire de manière optimale une fois qu'au moins le point de l'instrument sera identifié. De la même façon, l'unité de commande pourra contrôler la sonde pour la déplacer et/ou modifier sa position de référence et/ou modifier les capteurs de la sonde sollicités afin d'avoir la meilleure image possible d'au moins le point de l'instrument. Un volume réduit calculé autour d'un axe de la sonde à partir de l'image volumique pourra être exploité par la sonde pour augmenter la fréquence de rafraîchissement des images. De manière générale, l'unité de commande pourra ainsi être configurée pour optimiser le ou les images générées à l'écran.

Bien qu'ici l'unité de commande affiche à l'écran un message pour activer l'alarme si la distance déterminée dépasse un certain seuil (en étant supérieur ou inférieur à ce seuil), l'unité de commande pourra activer tout autre type d'alarme, appartenant ou non au dispositif, lorsque la distance dépassera un seuil donné.

Bien qu'on détermine le trajet virtuel via une ligne en trois dimensions représentant l'instrument déformé on pourra ne pas passer par cette ligne. On pourra ainsi identifier soit manuellement, soit automatiquement, sur au moins l'une des images un point d'insertion et/ou un point de la partie utile de l'instrument et/ou l'angle et/ou la déformation de l'instrument pour en déduire le trajet virtuel.

Le rafraîchissement automatique des images sera optionnel.

## Revendications

1. Dispositif de guidage d'un instrument médical comprenant :
- l'instrument médical,
- un appareil (2) de contrôle comportant un support (3) et une sonde (4) d'imagerie médicale qui est agencée sur le support et qui est destinée à être positionnée à proximité d'au moins une cible présente dans ou sur le patient,
- un écran (10), et
- une unité de commande (11) du dispositif qui est reliée à l'écran et à la sonde pour générer au moins une image en trois dimensions,
l'unité de commande étant configurée pour générer à l'écran à partir d'au moins ladite image en trois dimensions, au moins une image en deux dimensions montrant une déformation de l'instrument,
l'unité de commande étant configurée pour, à partir de cette déformation de l'instrument, estimer un trajet virtuel de l'instrument si on prolongeait son insertion jusqu'à la cible, et en déduire au moins une distance entre ledit trajet virtuel et la cible,
l'unité de commande (11) calculant, à partir de plusieurs points de l'instrument déformé sélectionnés sur l'image en trois dimensions et/ou sur l'image en deux dimensions, une ligne formée par l'instrument déformé,
l'unité de commande étant configurée pour, à partir de cette ligne, estimer le trajet virtuel de l'instrument.

2. Dispositif selon la revendication 1, dans lequel l'unité de commande (11) est configurée de manière à afficher sur une image en deux dimensions montrant une déformation de l'instrument, le trajet virtuel de l'instrument.

3. Dispositif selon l'une des revendications précédentes, dans lequel l'unité de commande (11) active une alarme lorsque la distance dépasse un seuil donné.

4. Dispositif selon la revendication 3, dans lequel l'unité de commande (11) affiche à l'écran (10) un message pour activer l'alarme.

5. Dispositif selon l'une des revendications précédentes, dans lequel au moins l'image en trois dimensions ou en deux dimensions est rafraîchie automatiquement.

6. Dispositif selon l'une des revendications précédentes, dans lequel le trajet virtuel est mis à jour automatiquement et/ou la distance entre ledit trajet virtuel et la cible est mise à jour automatiquement.

7. Dispositif selon l'une des revendications précédentes, dans lequel l'instrument est porté par l'appareil de contrôle (2).

8. Dispositif selon l'une des revendications précédentes, dans lequel l'instrument comporte une aiguille à biopsie (9) guidée par un porte-aiguille (5) de l'appareil de contrôle.

9. Dispositif selon l'une des revendications précédentes, dans lequel le dispositif comporte au moins deux instruments, le dispositif étant configuré de sorte que l'unité de commande (11) génère à l'écran :
- une première image en deux dimensions incluant un point du premier instrument et une deuxième image en deux dimensions incluant un point du deuxième instrument; et/ou
- une image en deux dimensions, incluant un point du premier instrument et un point du deuxième instrument.

10. Dispositif selon l'une des revendications précédentes, dans lequel l'image en deux dimensions montrant une déformation de l'instrument comprend au moins un point d'introduction de l'instrument dans le volume anatomique défini par l'image en trois dimensions, ledit point étant appelé « point d'insertion de l'instrument ».

11. Dispositif selon l'une des revendications précédentes, dans lequel l'image en deux dimensions montrant une déformation de l'instrument comprend au moins un point de l'instrument destiné à venir agir sur la cible, ledit point étant appelé « partie utile de l'instrument ».

12. Dispositif selon les revendications 10 et 11, dans lequel l'unité de commande (11) est configurée pour générer à l'écran à partir de ladite image en trois dimensions, au moins une image en deux dimensions présentant à la fois le point d'insertion et la partie utile de l'instrument.

13. Dispositif selon l'une des revendications précédentes, dans lequel l'unité de commande (11) est configurée pour générer à l'écran au moins une image en deux dimensions, ladite image en deux dimensions étant issue d'une réduction de l'image volumique à un plan par calcul d'une moyenne ou d'une somme d'amplitudes autour d'un axe de l'image volumique.

14. Dispositif selon l'une des revendications précédentes, dans lequel l'unité de commande (11) est agencée de sorte à piloter la sonde afin d'aligner une position de référence de la sonde avec celle de l'instrument.

## Patentansprüche

1. Vorrichtung zur Führung eines medizinischen Instruments, umfassend:
- das medizinische Instrument,
- ein Kontrollgerät (2), das einen Träger (3) und eine medizinische Abbildungssonde (4) umfasst, die auf dem Träger angeordnet ist und die dazu bestimmt ist, in der Nähe mindestens eines Ziels positioniert zu werden, das sich in oder auf dem Patienten befindet,
- einen Bildschirm (10) und
- eine Steuereinheit (11) zum Steuern der Vorrichtung, die mit dem Bildschirm und mit der Sonde verbunden ist, um mindestens ein dreidimensionales Bild zu erzeugen,
wobei die Steuereinheit ausgebildet ist, auf dem Bildschirm aus mindestens dem genannten dreidimensionalen Bild mindestens ein zweidimensionales Bild zu erzeugen, das eine Verformung des Instruments zeigt, wobei die Steuereinheit ausgebildet ist, anhand dieser Verformung des Instruments eine virtuelle Bahn des Instruments zu schätzen, wenn man seine Einführung bis zu dem Ziel verlängern würde, und daraus mindestens einen Abstand zwischen der genannten virtuellen Bahn und dem Ziel abzuleiten,
wobei die Steuereinheit (11) anhand mehrerer Punkte des verformten Instruments, die auf dem dreidimensionalen Bild und/oder auf dem zweidimensionalen Bild ausgewählt werden, eine von dem verformten Instrument gebildete Linie berechnet,
wobei die Steuereinheit ausgebildet ist, anhand dieser Linie die virtuelle Bahn des Instruments zu schätzen.

2. Vorrichtung nach Anspruch 1, bei der die Steuereinheit (11) so ausgebildet ist, dass sie auf einem zweidimensionalen Bild, das eine Verformung des Instruments zeigt, die virtuelle Bahn des Instruments anzeigt.

3. Vorrichtung nach einem der vorhergehenden Ansprüche, bei der die Steuereinheit (11) einen Alarm aktiviert, wenn der Abstand einen gegebenen Schwellenwert übersteigt.

4. Vorrichtung nach Anspruch 3, bei der die Steuereinheit (11) auf dem Bildschirm (10) eine Nachricht zum Aktivieren des Alarms anzeigt.

5. Vorrichtung nach einem der vorhergehenden Ansprüche, bei der mindestens das dreidimensionale oder das zweidimensionale Bild automatisch aktualisiert wird.

6. Vorrichtung nach einem der vorhergehenden Ansprüche, bei der die virtuelle Bahn automatisch aktualisiert wird und/oder der Abstand zwischen der genannten virtuellen Bahn und dem Ziel automatisch aktualisiert wird.

7. Vorrichtung nach einem der vorhergehenden Ansprüche, bei der das Instrument von dem Kontrollgerät (2) getragen wird.

8. Vorrichtung nach einem der vorhergehenden Ansprüche, bei der das Instrument eine Biopsienadel (9) umfasst, die von einem Nadelträger (5) des Kontrollgeräts geführt wird.

9. Vorrichtung nach einem der vorhergehenden Ansprüche, bei der die Vorrichtung mindestens zwei Instrumente umfasst, wobei die Vorrichtung derart ausgebildet ist, dass die Steuereinheit (11) auf dem Bildschirm erzeugt:
- ein erstes zweidimensionales Bild, das einen Punkt des ersten Instruments enthält, und ein zweites zweidimensionales Bild, das einen Punkt des zweiten Instruments enthält; und/oder
- ein zweidimensionales Bild, das einen Punkt des ersten Instruments und einen Punkt des zweiten Instruments enthält.

10. Vorrichtung nach einem der vorhergehenden Ansprüche, bei der das zweidimensionale Bild, das eine Verformung des Instruments zeigt, mindestens einen Einführungspunkt des Instruments in das anatomische Volumen umfasst, das von dem dreidimensionalen Bild definiert wird, wobei der genannte Punkt "Einführungspunkt des Instruments" genannt wird.

11. Vorrichtung nach einem der vorhergehenden Ansprüche, bei der das zweidimensionale Bild, das eine Verformung des Instruments zeigt, mindestens einen Punkt des Instruments umfasst, der dazu bestimmt ist, auf das Ziel einzuwirken, wobei der genannte Punkt "nützlicher Teil des Instruments" genannt wird.

12. Vorrichtung nach den Ansprüchen 10 und 11, bei der die Steuereinheit (11) ausgebildet ist, auf dem Bildschirm anhand des genannten dreidimensionalen Bildes mindestens ein zweidimensionales Bild zu erzeugen, das sowohl den Einführungspunkt als auch den nützlichen Teil des Instruments zeigt.

13. Vorrichtung nach einem der vorhergehenden Ansprüche, bei der die Steuereinheit (11) ausgebildet ist, auf dem Bildschirm mindestens ein zweidimensionales Bild zu erzeugen, wobei das genannte zweidimensionale Bild aus einer Reduzierung des Volumenbildes auf eine Ebene stammt, indem ein Mittelwert oder eine Summe von Amplituden um eine Achse des Volumenbildes berechnet wird.

14. Vorrichtung nach einem der vorhergehenden Ansprüche, bei der die Steuereinheit (11) derart ausgebildet ist, dass sie die Sonde steuert, um eine Referenzposition der Sonde mit der des Instruments auszurichten.

## Claims

1. A device for guiding a medical instrument, comprising:
- the medical instrument,
- a monitoring appliance (2) having a support (3) and a medical imaging probe (4), which is arranged on the support and which is intended to be positioned in proximity to at least one target present in or on the patient,
- a screen (10), and
- a control unit (11) which controls the device and which is connected to the screen and to the probe in order to generate at least one three-dimensional image, the control unit being configured to generate on the screen, from at least said three-dimensional image, at least one two-dimensional image showing a deformation of the instrument,
the control unit being configured to estimate, from this deformation of the instrument, a virtual path of the instrument if the insertion thereof were continued as far as the target, and to deduce therefrom at least one distance between said virtual path and the target,
the control unit (11) calculating, from several points of the deformed instrument that are selected on the three-dimensional image and/or on the two-dimensional image, a line formed by the deformed instrument,
the control unit being configured to use this line in order to estimate the virtual path of the instrument.

2. The device as claimed in claim 1, in which the control unit (11) is configured in such a way as to display the virtual path of the instrument on a two-dimensional image showing a deformation of the instrument.

3. The device as claimed in one of the preceding claims, in which the control unit (11) activates an alarm when the distance exceeds a given threshold.

4. The device as claimed in claim 3, in which the control unit (11) displays on the screen (10) a message for activating the alarm.

5. The device as claimed in one of the preceding claims, in which at least the three-dimensional or two-dimensional image is refreshed automatically.

6. The device as claimed in one of the preceding claims, in which the virtual path is updated automatically and/or the distance between said virtual path and the target is updated automatically.

7. The device as claimed in one of the preceding claims, in which the instrument is carried by the monitoring appliance (2).

8. The device as claimed in one of the preceding claims, in which the instrument has a biopsy needle (9) guided by a needle holder (5) of the monitoring appliance.

9. The device as claimed in one of the preceding claims, in which the device has at least two instruments, the device being configured in such a way that the control unit (11) generates on the screen (10):
- a first two-dimensional image including a point of the first instrument and a second two-dimensional image including a point of the second instrument; and/or
- a two-dimensional image including a point of the first instrument and a point of the second instrument.

10. The device as claimed in one of the preceding claims, in which the two-dimensional image showing a deformation of the instrument comprises at least one point of introduction of the instrument into the anatomical volume defined by the three-dimensional image, said point being called the "point of insertion of the instrument".

11. The device as claimed in one of the preceding claims, in which the two-dimensional image showing a deformation of the instrument comprises at least one point of the instrument intended to act on the target, said point being called the "useful part of the instrument".

12. The device as claimed in claims 10 and 11, in which the control unit (11) is configured to generate on the screen, from said three-dimensional image, at least one two-dimensional image presenting both the point of insertion and the useful part of the instrument.

13. The device as claimed in one of the preceding claims, in which the control unit (11) is configured to generate on the screen at least one two-dimensional image, said two-dimensional image being derived from a reduction of the volume image to a plane by calculation of an average or of a sum of amplitudes around an axis of the volume image.

14. The device as claimed in one of the preceding claims, in which the control unit (11) is arranged in such a way as to steer the probe in order to align a reference position of the probe with that of the instrument.
